# EUROPEAN PATENT APPLICATION

(11) **EP 2 281 814 A1**
(43) Date of publication of application: **09.02.2011**
(21) Application number: 09176520.6
(22) Date of filing: 19.11.2009
(51) Int. Cl.: C07C 327/46, C07C 231/02

(54) **A continuous acetylation process in the synthesis of non-ionic X-ray contrast agents**

(30) Priority: 21.07.2009 US 227091 P
(71) Applicant: GE Healthcare AS, 0401 Oslo (NO)
(72) Inventor: Cervenka, Jan, 0401 Oslo (NO); Hussain, Khalid, 0401 Oslo (NO)
(74) Representative: Wulff, Marianne Weiby

(57) **Abstract**

This invention relates to a method for the synthesis of 5-acetamido-N,N'-bis(2,3-dihydroxypropyl)-2,4,6-triiodoisophthalamide ("Compound A"), an intermediate in the industrial preparation of non-ionic X-ray contrast agents. In particular, it relates to a continuous process of acetylation of 5-amino-N,N'-bis(2,3-dihydroxypropyl)-2,4,6-triiodo-1,3-benzenedicarboxamide ("Compound B") followed by the removal of acetic anhydride.

## Description

### TECHNICAL FIELD

This invention relates generally to large-scale synthesis of non-ionic X-ray contrast agents. It further relates to an improved method for the synthesis of 5-acetamido-N,N'-bis(2,3-dihydroxypropyl)-2,4,6-triiodoisophthalamide ("Compound A"), an intermediate in the industrial preparation of non-ionic X-ray contrast agents. In particular, it relates to a continuous process for acetylation and the subsequent removal of the excess acetylation reagent.

### BACKGROUND OF THE INVENTION

Non-ionic X-ray contrast agents constitute a very important class of pharmaceutical compounds produced in large quantities. 5-[N-(2,3-dihydroxypropyl)-acetamido]-N,N'-bis(2,3-dihydroxypropyl)-2,4,6-triiodo-isophthalamide ("iohexol"), 5-[N-(2-hydroxy-3-methoxypropyl)acetamido]-N,N'-bis(2,3-dihydroxypropyl)-2,4,6-triiodo-isophthalamide ("iopentol") and 1,3-bis(acetamido)-N,N'-bis[3,5-bis(2,3-dihydroxypropyl-aminocarbonyl)-2,4,6-triiodophenyl]-2-hydroxypropane ("iodixanol") are important examples of such compounds. They generally contain one or two triiodinated benzene rings.

For example, iodixanol, marketed under the trade name Visipaque®, is one of the most used agents in diagnostic X-ray procedures. It is produced in large quantities by GE Healthcare in Lindesnes, Norway. The industrial production of iodixanol involves a multistep chemical synthesis as shown in Scheme 1 below. *See also* U.S. Patent No. 6,974,882. To reduce the cost of the final product, it is critical to optimize each synthetic step. Even a small improvement in reaction design can lead to significant savings in a large scale production.

In the acetylation step of the industrial scale synthesis 5-amino-N,N'-bis(2,3-dihydroxypropyl)-2,4,6-triiodoisophthalamide (Compound B), is acetylated to produce 5-acetylamino-N,N'-bis(2,3-dihydroxypropyl)-2,4,6-triiodoisophthalamide (Compound A) using acetic anhydride as the acetylating reagent. The instant invention is directed to a continuous process for acetylation of Compound B and the subsequent removal of the excess acetic anhydride.

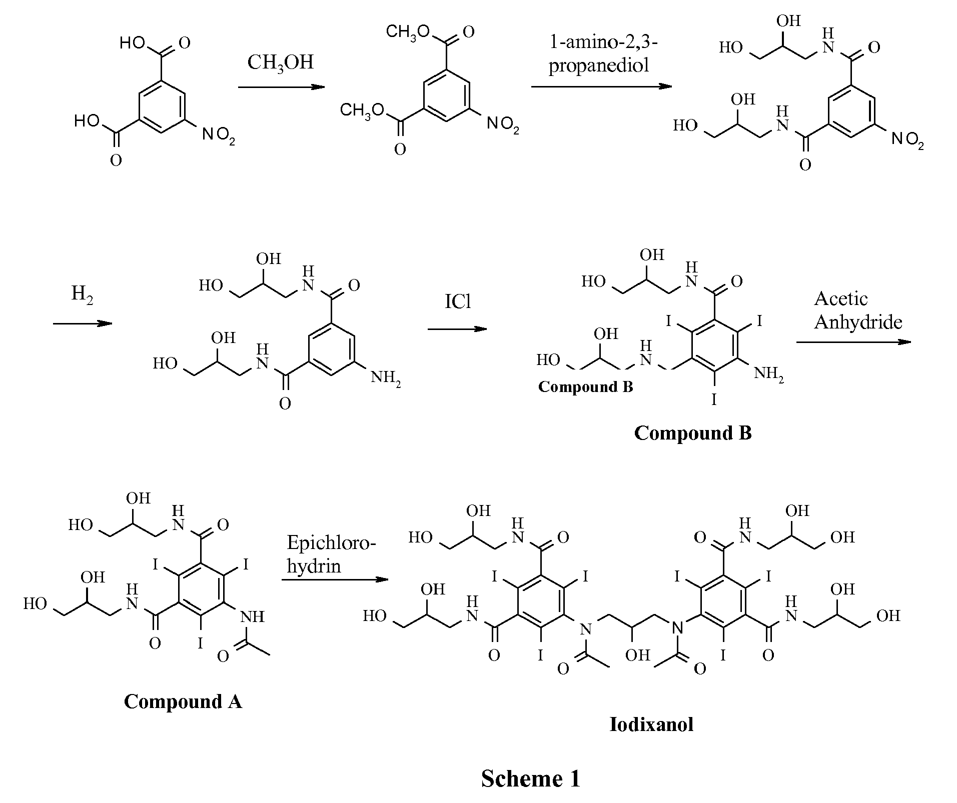

### BRIEF DESCRIPTION OF THE DRAWINGS

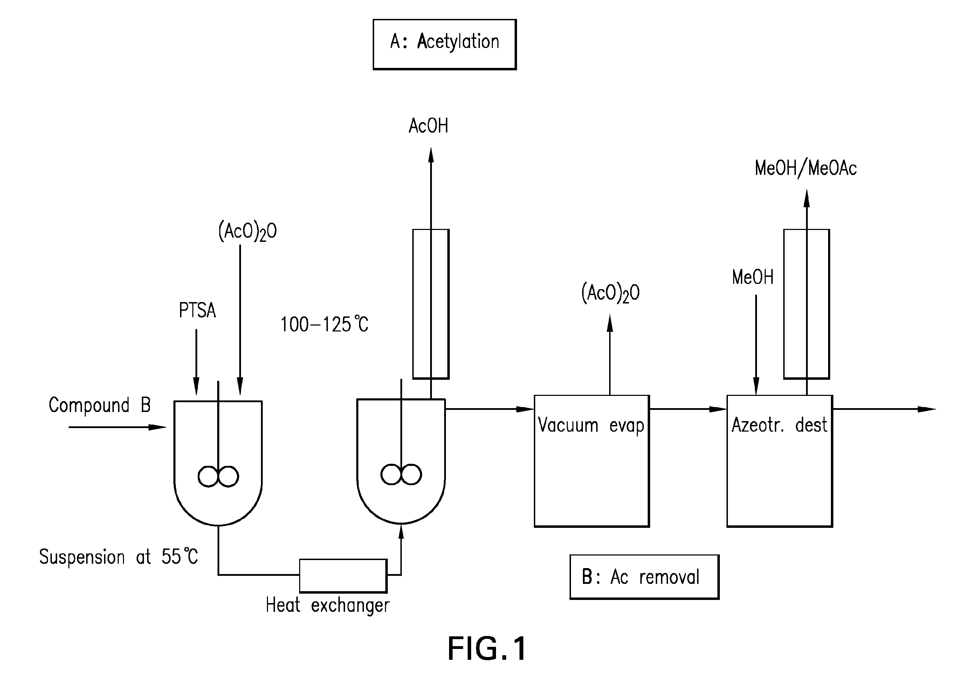

FIG. 1 is a schematic diagram of one exemplary embodiment of the instant invention.

### SUMMARY OF THE INVENTION

The present invention provides an industrial process for producing Compound A. Specifically, it uses a continuous process comprising the steps of (1) acetylating 5-amino-N,N'-bis(2,3-dihydroxypropyl)-2,4,6-triiodoisophthalamide ("Compound B") in a first continuous stirred tank reactor at a temperature between about 40 °C and about 70 °C; (2) acetylating Compound B in a second continuous stirred tank reactor at a temperature between about 70 °C and 125 °C; (3) removing acetic anhydride and acetic acid under a pressure of between about 50 mbar and about 150 mbar and at a temperature of between about 70 °C and about 100 °C; and (4) adding methanol and simultaneously distilling off a mixture of methanol and methyl acetate at atmospheric pressure.

An alternative continuous process of the instant invention comprises the steps of (1) acetylating Compound B in a tubular reactor wherein the reaction begins at a temperature between about 40 °C and about 70 °C and ends at a temperature between about 70 °C and 125 °C; (2) removing acetic anhydride and acetic acid under a pressure of between about 50 mbar and about 150 mbar and at a temperature of between about 70 °C and about 100 °C; and (3) adding methanol and simultaneously distilling off a mixture of methanol and methyl acetate at atmospheric pressure.

### DETAILED DESCRIPTION OF THE INVENTION

In a batch process, all the operations are performed successively in the same reactor. Thus, production capacity increase in a batch production requires very large reactor volumes and a corresponding increase in capital investments. On the other hand, a continuous process has dedicated equipment for every operation, where the mixture moves from one operation to the next within the production line. All the operations are performed continuously all the time and the system is at a steady state. Consequently, a continuous production process requires much smaller equipment volumes and investments for achieving the same production capacity. In addition, a continuous operation allows for less varying quality of the product. While desirable, a continuous system is more complicated to design and highly specific to one single product and one production rate.

In the present invention, we have found that the process of acetylation and the removal of excess acetic anhydride can be run continuously using a number of sequential steps. First, the acetylation reaction is a heterogeneous reaction where the limited soluble start material (Compound B) dissolves when acetylated. The reaction is also strongly exothermic and therefore the temperature control is essential.

In one embodiment of the instant process, two continuous stirred tank reactors (CSTRs) are used. In the first CSTR, acetylation is carried out at a temperature of about 40 °C to about 70°C, preferably at about 50 °C to about 65 °C. In the second CSTR, acetylation is carried out at about 70 °C to about 125 °C, preferably at about 100 °C to about 120 °C. A distillation tower may be operatively connected to the second CSTR for the purpose of minimizing the excess of acetic anhydride. It is also possible to distill off acetic anhydride/acetic acid without the distillation tower. The acetic acid (a byproduct of the acetylation reaction) is continuously distilled off through the distillation tower. In particular, the boiling point of acetic acid is 19 °C lower than that of acetic anhydride with no azeotrope. The temperature of the system can be effectively controlled by a cooling jacket, cooling coils, an external cooling loop or by boiling under reduced pressure. At the steady state, a very low acetic acid concentration can be kept.

Alternatively, the acetylation reaction may be conducted in a tubular reactor where it may be divided into two sections: an initial heating section and a cooling section to control the reaction temperature. In a tubular flow design, the acetylation reaction may start at a temperature between about 40 °C and about 70 °C and end at a temperature between about 70 °C and 125 °C.

Second, with respect to the acetic anhydride removal step, the instant continuous process involves removing acetic anhydride and acetic acid under a pressure of between about 50 mbar and about 150 mbar and at a temperature of between about 70 °C and about 100 °C. The resulting concentration of overacetylated Compound A in the output from this step is typically as high as the physical conditions (e.g., the viscosity of the solution) allows. For example, the concentration may be reduced down to about one liter reaction mixture per kilogram of Compound B added to the first reactor at steady state.

Next, methanol is added and, simultaneously, a mixture of methanol and methyl acetate is distilled off at atmospheric pressure. The purpose of adding methanol (b.p. 65 °C) is to esterify the residual acetic anhydride that remains after the distillation of acetic acid. The methylacetate formed is distilled off as an azeotrope with methanol (b.p.57 °C, 81 w % MeOAc). In this step, the high boiling point acetic anhydride is converted to a low boiling derivative (methylacetate), which is easy to remove without disturbing the remaining reaction mixture. The esterification of acetic anhydride is also exothermic and causes control problems in the batch mode. These problems are addressed in a continuous mode by simultaneous distillation of methanol and methyl acetate, while adding methanol.

The invention is illustrated further by the following examples that are not to be construed as limiting the invention in scope to the specific procedures described in them.

### EXAMPLES

### EXAMPLE 1

Compound B is suspended in a mixture of acetic anhydride and acetic acid (5-15 v/v % acetic acid, about 1.5-3.0 liter of acetic anhydride and acetic acid combined per kg of Compound B) in a continuous facility as described in Figure 1. The acetylation is performed in two steps in continuous stirred tank reactors at different temperatures (55 and 120°C) with residence times from about 0 to 2 hours. Excess acetic acid and acetic anhydride are removed by distillation in a continuous evaporation system. The viscous residue is diluted with methanol (which reacts with residual acetic anhydride and also with O-acetyl groups deriving from Compound B), and the resulting methyl acetate is continuously removed by atmospheric distillation at about 50-60°C. The HPLC purity of the resulting crude reaction mixture is about 98 % with respect to Compound A.

All patents, journal articles, publications and other documents discussed and/or cited above are hereby incorporated by reference.

## Claims

1. A process for industrial preparation of 5-acetamido-N,N'-bis(2,3-dihydroxypropyl)-2,4,6-triiodoisophthalamide ("Compound A") comprising the following sequential and continuous steps:
(1) acetylating 5-amino-N,N'-bis(2,3-dihydroxypropyl)-2,4,6-triiodoisophthalamide ("Compound B") in a first continuous stirred tank reactor at a temperature between about 40 °C and about 70 °C;
(2) acetylating Compound B in a second continuous stirred tank reactor at a temperature between about 70 °C and 125 °C;
(3) removing acetic anhydride and acetic acid under a pressure of between about 50 mbar and about 150 mbar and at a temperature of between about 70 °C and about 100 °C; and
(4) adding methanol and simultaneously distilling off a mixture of methanol and methyl acetate at atmospheric pressure.

2. A process for industrial preparation of 5-acetamido-N,N'-bis(2,3-dihydroxypropyl)-2,4,6-triiodoisophthalamide ("Compound A") comprising the following sequential and continuous steps:
(1) acetylating 5-amino-N,N'-bis(2,3-dihydroxypropyl)-2,4,6-triiodoisophthalamide ("Compound B") in a tubular reactor wherein the reaction begins at a temperature between about 40 °C and about 70 °C and ends at a temperature between about 70 °C and 125 °C;
(2) removing acetic anhydride and acetic acid under a pressure of between about 50 mbar and about 150 mbar and at a temperature of between about 70 °C and about 100 °C; and
(3) adding methanol and simultaneously distilling off a mixture of methanol and methyl acetate at atmospheric pressure.
